# EUROPEAN PATENT APPLICATION

(11) **EP 4 349 255 A1**
(43) Date of publication of application: **10.04.2024**
(21) Application number: 22200188.5
(22) Date of filing: 07.10.2022
(51) Int. Cl.: A61B 5/11, A61B 5/00

(54) **WRITING INSTRUMENT**

(71) Applicant: BIC Violex Single Member S.A., 145 69 Anoixi (GR)
(72) Inventor: Chrysanthakopoulos, Nikolaos, 145 69 Anoixi (GR); Antonakis, Ion - Ioannis, 145 69 Anoixi (GR)
(74) Representative: Peterreins Schley

(57) **Abstract**

The present disclosure relates to a computer-implemented method for monitoring a writing tip force of a writing instrument, comprising:
monitoring the force between the writing tip of the writing instrument and a writing surface during a writing session with the writing instrument;
generating data from the monitored force;
storing generated data as historical data
comparing the generated data to historical data from the user; and
providing an indication in case that data of at least two writing sessions of the user deviates from an average of historical data from the user.

## Description

### Technical Field

The present disclosure relates to a method for monitoring a writing tip force of a writing instrument's user, a writing instrument for monitoring a writing tip force of its user, and a digital surface device for monitoring a writing tip force of its user.

### Background

A large study attempted to associate drawing features with Montreal Cognitive Assessment (MoCA) scores for assessment of global cognition from a plurality of adults. It was shown that adults with low MoCA scores (potential of Cognitive Disorders) were having higher pause to drawing duration ratios while also varying the pressure applied to the writing surface (pen tip pressure on the surface). Therefore, there is a correlation between the two and the potential of a user having or developing a Cognitive Disorder.

Another study with a plurality participants assessed the kinematic and pressure features of handwriting and drawing using a computerized system and attempted to create potential correlations between their results and the possibility of the participant having developed Alzheimer Disease. The study indicated that pen pressure on paper and the time between consecutive strokes while writing (pauses on air) can be used to indicate if the user could be suffering from AD or MCI, while the combination of that data with the acceleration and directionality of the stroke can further improve the results.

A review article that is focusing on the influence of neurodegenerative diseases on handwriting concluded that healthy normal writing instrument users, put significantly more tension on the pen while writing (a difference of more than 10%). Moreover, they indicated that in contrast to healthy normal participants, subjects with MCI and Mild AD had significantly longer time in the air while writing. Further supporting the claims from the aforementioned studies.

Last but not least, researchers are creating an Experimental Protocol to Support Cognitive Impairment Diagnosis by using Handwriting Analysis. In this protocol, pen pressure and "time in the air" while writing are included as valuable metrics for determining if a user has the potential to develop a Cognitive Disorder.

Alzheimer's Disease (AD) is a form of dementia, a neurodegenerative disease of the brain. The disease process is associated with amyloid plaques, neurofibrillary tangles, and loss of neuronal connections in the brain. Unfortunately, the cause of Alzheimer's disease is poorly understood. There are many environmental and genetic risk factors associated with it. The strongest genetic risk factor is from an allele of APOE gene. Other risk factors are history of head injury, clinical depression, and high blood pressure.

Mild Cognitive Impairment (MCI) is an early stage of memory loss or other cognitive ability loss (such as language or visual/spatial perception) in individuals who maintain the ability to independently perform most daily activities. Individuals with MCI may have a higher risk of developing dementia.

### Summary

In a first general aspect, the present disclosure relates to a computer-implemented method for monitoring a writing tip force of a writing instrument, comprising:
monitoring the force between the writing tip of the writing instrument and a writing surface during a writing session with the writing instrument;
generating data from the monitored force;
storing generated data as historical data
comparing the generated data to historical data from the user; and
providing an indication in case that data of at least two writing sessions of the user deviates from an average of historical data from the user.

In embodiments, the method may comprise storing monitored pressure data as historical pressure data.

In embodiments, the method may comprise extrapolating a time span of the writing instrument not in contact with the writing surface between two consecutive writing strokes from the monitored force; and generating data from the monitored force and the extrapolated time span. In embodiments, at least one step of the method may be executed by a digital surface device comprising the writing surface.

In embodiments, an indication may be provided in case the force is below an average force of historical data and/or a time span of the writing instrument not in contact with the writing surface between two consecutive writing strokes is above an average time span of historical data.

In embodiments, the method may comprise providing personal user data for calibration and/or comparing the generated data to historical data from the user.

In embodiments, the method may comprise alerting the user in case of an indication by activating a tactile feedback generator of the writing instrument.

In embodiments, the indication may be provided via a user interface of the writing instrument or wherein the indication is provided via a wireless communication system to an external device having an interface.

In a second general aspect, the present disclosure relates to a writing instrument for monitoring a writing tip force of its user, comprising:
one or more sensors configured to monitor the force between the writing tip of the writing instrument and a writing surface during a writing session with the writing instrument;
a communication system configured to provide an indication in case that data of at least two writing sessions of the user deviates from an average of historical data from the user;
a storage system configured to store generated data as historical data; and
   - computer system configured to execute the computer-implemented method for monitoring a writing tip force of a digital writing instrument as described above.

In embodiments, the writing instrument may comprise a hand presence sensor configured to obtain information regarding a usage state of the writing instrument.

In embodiments, the writing instrument may comprise a tactile feedback generator configured to alert the user in case that data of at least two writing sessions of the user deviates from an average of historical data from the user.

In embodiments, the writing instrument may comprise an ink cartridge comprising a tip end and an opposed end, wherein the one or more sensors are mechanically connected to the ink cartridge.

In embodiments, the writing instrument may comprise a barrel-shaped housing, wherein the one or more sensors are arranged between the opposed end of the ink cartridge and the housing.

In embodiments, the writing instrument may comprise a barrel-shaped housing, wherein the one or more sensors comprise a ring of a material that can convert mechanical pressure to an electrical signal and wherein the ring is arranged between the ink cartridge and the housing.

In a third general aspect, the present disclosure relates to a digital surface device for monitoring a writing tip force of its user, comprising:
a writing surface configured to be written upon by a writing instrument;
one or more sensors configured to monitor the force between the writing tip of the writing instrument and the writing surface during a writing session with the writing instrument;
a communication system configured to provide an indication in case that data of at least two writing sessions of the user deviates from an average of historical data from the user; and
a computer system configured to execute the computer-implemented method for monitoring a writing tip force of a digital writing instrument se described above.

In embodiments, a user is enabled to be notified over the potential of developing a cognitive disorder via the use of smart writing instrument with pen tip pressure sensors. This is enabled by a system on the pen that monitors and tracks the applied pressure on the writing surface and also their time between consecutive writing strokes i.e., air-time over time.

In embodiments, force sensors on specific pen body areas that come in constant contact with writing instrument's ink cartridge measure the applied force of the pen's nib on the writing surface over time and over specific tasks and compare it with user's historical data. Further, that data may be used to extract the time that the user is not contacting the writing surface while they are writing or drawing i.e., air-time. If the force data captured deviate significantly, the user is alerted.

In embodiments, the user initiates their note taking process as usual. As they write down their notes or everyday tasks, the pen monitors the force that they apply with their pen on their writing surface as well as their air-time. This is achieved by placing force sensors between the pen barrel and the ink cartridge in multiple locations e.g., the end of the ink cartridge and the rear of the pen's barrel. Said sensors can capture the force data with a fairly high accuracy over a wide range in order to ensure that any variances can be identified. The signals from the sensors are transferred to and analyzed by the on-board electronics.

In embodiments, the user is alerted by a mobile device connected to the smart pen or alternatively by a tactile feedback mechanism on the pen. Tactile feedback mechanism can generate frequencies below 500Hz and is located as close to the tip of the writing instrument as possible, to ensure that the vibrations are felt by the user's finger tips. The fingertips are sensitive enough that can differentiate and identify a wide range of vibration frequencies.

In embodiments, the device also incorporates the required electronics and software drivers for connecting wirelessly e.g., Bluetooth, Wi-Fi, etc. to an optional supplementary device.

In embodiments, the system can now in real time, capture the pen's tip pressure and compare that data to previous known historical data on that specific user's average applied pressure on the writing surface. Additionally, the device can extrapolate the current air-time and compare it to historical data. If the current extracted writing features deviate significantly from the historical average, for multiple sessions, the writing instrument will notify the user by a mobile device app alert or via a series of tactile vibrations on the pen. This alert will signal the user to go and seek medical attention.

An example system of the present disclosure may provide the following features:
A digital writing instrument with force sensors that can capture the pen nib tip pressure applied by the user during its use.
A storage medium and system for storing the historical tip pressure data over the devices lifetime.
A sensor on the pen body at the finger grip position detecting that the user holds the pen with his fingers.
An algorithm responsible for comparing the current writing pen tip's pressure data data to the historical ones and hence providing an indication to the user in the event that there is a large data variance (which could be associated with dementia or cognitive disorders).
An algorithm that can extract the user's air-time from the pen pressure data and complement the system's ability to detect the potential of a cognitive disorder development.
A tactile feedback generator that can alert the user.

Particular examples of the first to third general aspects can be implemented so as to realize one or more of the following advantages.

First, a system is provided for enabling the user to be notified on the potential of developing a cognitive disorder e.g., AD, MCI, etc. and seek medical attention to confirm the findings. The writing instrument is a wellness device.

Second, the present disclosure enables a writing instrument such as a personal smart pen to constantly monitor the user's applied pressure on the writing surface via the pen's nib while they are writing or drawing. Simultaneously, that data is used to extrapolate the air-time between consequent strokes. As the user is taking notes or performing tasks that require writing, over time, the device is creating a profile that can be used as reference.

Third, if the pen tip pressure varies significantly i.e., is reduced and/or their air-time is increased for multiple writing and/or drawing sessions, the pen can notify them and indicate the need for medical attention to confirm or deny the potential development of a cognitive disorder. Said smart pen system is always adapting to the user's current state and does not need any additional maintenance.

Regarding pressure or force sensors, piezoelectric crystals (e.g cellulose microfiber or flexible liquid metal-tin sulfide) may be employed, that can deliver high electrical throughput and power density under repeated mechanical stress as for example in the case of hand punching for portable items to power low-power electronics. These PE nanogenerators can generate voltage in the order of milli-Volts (50-120mV) with a power output of several micro-Watts. Said materials can be used for measuring the force applied by the user, as their voltage output is related to the pressure applied.

In embodiments, monitoring the force between the writing tip of the writing instrument and the writing surface during a writing session is performed with a writing instrument configured to monitor a tip force that comprises two or more magnets housed inside the barrel shaped body of the writing instrument. A first magnet may be arranged at the end of the tip of the writing instrument that is concealed inside the barrel shaped body. The first magnet exhibits a magnetic moment which can be measured e.g. by magnetometers arranged on the digital surface device. The first magnet is configured to move translationally along a longitudinal axis upon pressure exerted by the tip of the writing instrument during a writing session. The second magnet of the pair of magnets is permanently fixed to a wall inside the barrel shaped body of the writing instrument at a distance that is sufficiently far from the first magnet so as to ensure the first magnet does not come in contact with the second magnet when the tip is in a retracted position. Such a configuration of the first and second magnets is described in document US2014362057A1, its content being herein incorporated by reference in its entirety.

In embodiments, the digital surface device may comprise a plurality of magnetometers that are mechanically linked to one another without any degree of freedom so as to preserve a predefined distance between each of said magnetometers. Such a configuration of the first and second magnets and magnetometers is briefly described in document US2014362057A1, its content being herein incorporated by reference in its entirety. The digital surface device may also comprise a communication system configured to provide an indication in case that data of at least two writing sessions of the user deviates from an average of historical data from the user; and a computer system configured to execute a computer-implemented method for monitoring a writing tip force of a digital writing instrument substantially as described herein.

With the rise in sales of portable devices and more especially wearables, and the consumer need for thinner and smaller devices, haptic and tactile feedback technologies have been miniaturized to the degree that they can fit in a small form-factor device. Such devices range from vibration motors to linear actuators and piezoelectric crystals, which can accomplish complex haptic sensations with a low-power requirements.

Regarding writing action, handwriting is the writing action done with a writing instrument, such as a pen or pencil, using the hand. Handwriting includes both printing and cursive styles. Handwriting involves the use of a pen or pencil which the user keeps holding with his hand/fingers even when he does not write.

The normal pen grip may be performed in the following way:
- the writing instrument is held in a stable position between the thumb, index and middle fingers,
- the ring and little fingers are bent and rest comfortably on the table,
- the index finger and thumb form an open space,
- the wrist is bent back slightly, and the forearm is resting on the table,
- the writing instrument is held about 1- 2 cm from the tip.

So, especially while writing, a user exerts a gripping force on the body of the pen.

Certain terms are used in the following manner in the present disclosure:
The expression "Writing Instrument Barrel" may refer to a main body of the writing instrument that provides the core structure of the pen and comes in direct contact with the user's fingertips.

The expression "digital device" may refer to an electronic device that uses discrete, numerable data and processes for all its operations, and is capable of displaying content to the user. Examples of such a device include but are not limited to: Mobile phones, Laptops, Tablets, Personal computers, Netbooks, iPads, etc.

The expression "haptic or tactile feedback" may refer to a physical response on a digital device from the user input, also known as kinaesthetic communication. The application with mechanical or electronic means of force, vibrations, or motions to the user in response to a user's action on the digital device.

The expression "human machine interface" may refer to a user interface or dashboard or an input system that connects a person to a machine, system, or device. For example, a physical button on a remote controller.

The expression "Handwriting air-time" may refer to the time spent of the writing instrument not in contact with the writing surface between two consecutive writing strokes e.g., the time spent between writing each letter.

The expression "group of users" may refer to a panel of users selected through specific methodology, with the scope to test products and provide feedback, in order to provide statistical insights to specific parameters of the products and their respective use.

### Description of the Drawings

Fig. 1 illustrates an overview of components of a writing instrument according to the present disclosure.
Fig. 2 illustrates a process flow diagram of a writing instrument according to the present disclosure.
Fig. 3 illustrates a perspective view of a writing instrument according to the present disclosure.
Fig. 4 illustrates a perspective view of a writing instrument according to the present disclosure.
Fig. 5 illustrates a perspective view of a writing instrument according to the present disclosure.
Fig. 6 illustrates a method flow chart for monitoring a fingertip strength of a writing instrument's user according to the present disclosure.

### Detailed Description

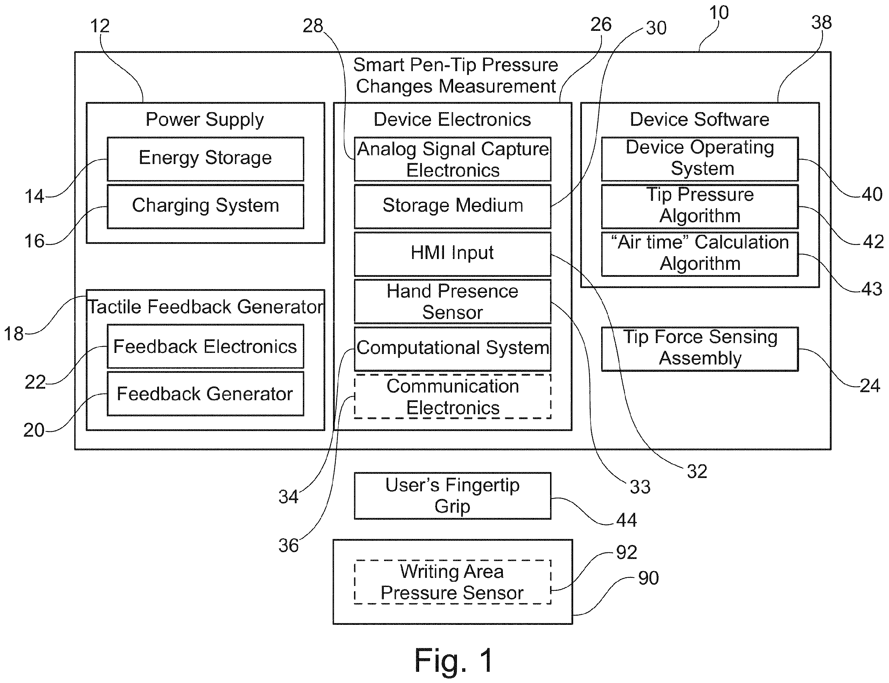

**Fig. 1** shows an overview of components of a writing instrument 10 according to the present disclosure. The writing instrument 10 can be named Tip Pressure Changes Measurement Smart Pen, digital writing instrument or digital device.

An example of the writing instrument 10 enables the user to monitor their applied pressure on the writing surface via the pen tip while they are using the writing instrument 10 as well as their air-time, and alert them in the event that there is significant variance in the measurements, which could indicate the potential of a cognitive disorder.

As shown in **Fig. 1**, the writing instrument 10 includes a power supply 12 to provide the energy for the following sub-systems of the writing instrument 10. The power supply 12 includes a subsystem energy storage 14 which is the energy source of the writing instrument 10 and can be of the form of a battery. The power supply 12 includes a subsystem charging system 16 which includes the electronics for charging and maintaining the health of the energy storage 14.

The writing instrument 10 includes a subsystem tactile feedback generator 18 that is capable of receiving digital data and converting them into vibrations that can be felt by the user. Hence, its location can be as close to the user's finger tips as possible i.e., close to the tip of a digital pen.

The tactile feedback generator 18 includes a subsystem feedback generator 20 that is responsible for converting the analog electrical signals i.e., electrical energy into mechanical energy in the form of vibrations that can be felt by the user i.e., the user's fingertips. The subsystem may generate vibrations in the range of 0 to 500 Hz with a controllable amplitude, while being power efficient enough to be integrated within a portable consumer product.

The feedback generator 20 can be of the form but not limited to at least one of rotary motors with a weight off the center of rotation, linear vibration motors, and piezoelectric vibration motors.

The tactile feedback generator 18 may include feedback electronics 22 that include the hardware i.e., motor drivers and firmware for converting the digital signal e.g., in the form of binary into an electrical e.g., analog signal that can drive the feedback generator 20.

The writing instrument 10 includes a subassembly tip force sensing assembly 24 that encompasses the hardware required for converting mechanical pressure into an electrical signal.

In examples of the present disclosure, this subassembly is located at the grip of the writing instrument 10 i.e., the location where the user usually rests and grips the pen with his fingers during their writing.

The type of the force sensors could be of the form but not limited to at least one of:
i. piezoelectric materials
ii. strain gauges
iii. load cells
iv. optical fiber force sensors
v. optical sensors
vi. induction sensors
vii. piezoelectric (PE) nanogenerators

The writing instrument 10 includes a component writing instrument electronics or device electronics 26 which enables the writing instrument 10 to function.

The device electronics 26 include the subcomponent analog signal capture electronics 28 that encompasses all electronics including the physical and digital signal filters and the analog to digital converter A2D as well as firmware to capture an analog signal from the tip force sensing assembly 24 and convert it into a digital format.

The device electronics 26 may include a subcomponent storage medium 30 that includes the hardware, firmware and software for the writing instrument 10 to store data captured by the system and more specifically the analog signal capture electronics 28. Moreover, this subcomponent allows the writing instrument operating system or device operating system 40 to load said data and utilize it for a wide range of uses e.g., data comparison, data viewing, data deletion, etc.

The storage medium 30 could be of the form but not limited to at least one of magnetic storage, optical storage, and solid state storage (e.g., NAND Flash, NOR Flash, etc.).

The device electronics 26 may include a subsystem human machine interface (HMI) input 32 that includes all of the hardware and software for the writing instrument 10 to accept human input and display its state to the user. The physical input interface of the writing instrument 10 could be of the form of a button, a rotational encoder wheel, or a linear slider. The display element of this interface can be at least one LED light of single or multiple colors. The input and display interfaces are read and controlled by the device operating system 40.

The device electronics 26 may include a subcomponent hand presence sensor 33 that gives feedback to the device regarding its usage state i.e., if it is being held or used or if it is resting on a surface. The hand presence sensor 33 is located on the pen barrel close to the area that the user is making direct contact with their fingertips. Said information can be used to enhance the accuracy of the algorithm that calculates the user's air-time and/or if the device should enter a "sleep" mode.

The sensor used can be of the form of at least one of capacitive sensors, optical proximity sensors, ultrasonic proximity sensors, time of flight sensors, and piezoelectric sensors.

The device electronics 26 may include a subsystem computational system 34 that includes the hardware for the writing instrument 10 to function. The main function of this subsystem is to run the device operating system 40 and coordinate the hardware of the writing instrument 10 such that the user's requests and inputs to the writing instrument 10 are recognized and executed accordingly. In addition, it enables the writing instrument's 10 functionality via executing the writing instrument's 10 main software regarding the storage medium 30 and the human machine interface (HMI) input 32. Said subsystem include the Central Processing Unit (CPU), as well as the Random-Access Memory (RAM) and Read Only Memory (ROM).

The device electronics 26 may include a subcomponent communication electronics 36 for establishing a wireless connection between the digital writing instrument 10 and other devices such as a digital device, a mobile phone, a personal computer, etc.). The communication protocol could be of the form but not limited to at least one of Bluetooth, NFC, and Wireless Fidelity (WiFi).

The communication electronics 36 may include or may be in communication with an HMI display component of the writing instrument 10. It should be noted that the HMI display component can be used for displaying relevant information to the user such as at least one of the writing instrument's 10 status (ON or OFF), the selected vibration pattern's intensity (e.g., blue color = low amplitude, red = high/maximum amplitude), etc.

Building upon the notification system of the feedback electronics 22, it is possible to utilize the optional communication electronics 36 on the smart pen device and notify the user of their reduction in hand grip strength via an application on their mobile phone. Additionally, this system may enable the user to visually see a graph of their measurements over time or even the raw captured data.

The writing instrument 10 includes a subcomponent device software 38.

The subcomponent device software 38 includes a software component device operating system 40 also known as Operating System (OS) that manages the writing instrument's 10 hardware, software resources, and provides common services for application specific algorithms.

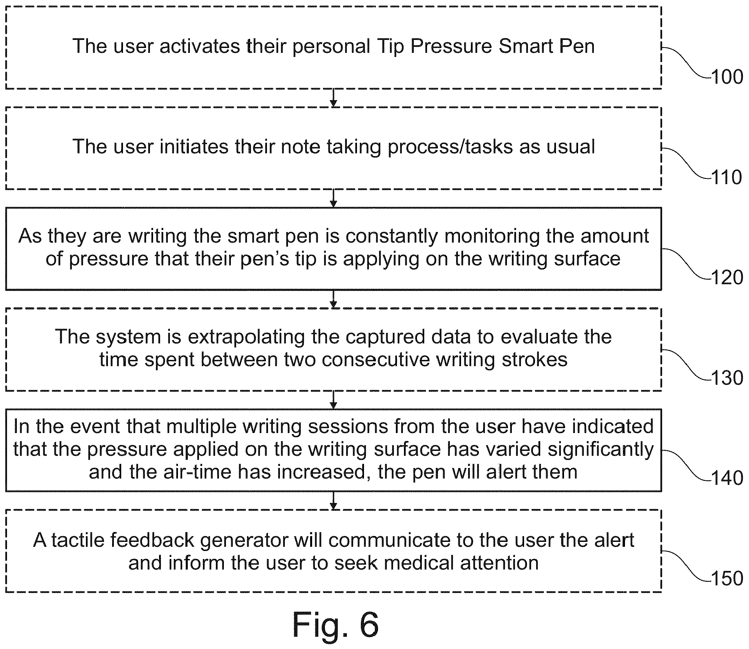

The subcomponent device software 38 may include a tip pressure algorithm 42 that is responsible for utilizing the data from a tip force sensing assembly and generating a value that is related to the user's applied force on the writing surface via the pen's nib in Newton. Said algorithm is running in the background constantly and comparing its currently generated value to historical data that are read from the storage medium 30. Said algorithm may include or consist of the method for monitoring a writing tip force of a writing instrument's user an example of which is depicted in Fig. 6.

The subcomponent device software 38 may include an "air-time" calculation algorithm 43 that is responsible for determining the amount of time the user is spending without contacting the writing surface between two consecutive writing strokes. Said algorithm is making use of the data supplied by the tip force sensing assembly. Hence, the data of the tip force sensing assembly in addition to the data from the hand presence sensor 33 is utilized in order to ensure that the user is actually engaging with the writing instrument 10 and it is not left on a surface for example.

The algorithm is looking at the absolute signal from the tip force sensing assembly over time and it is assuming that the at point of contact the forces will spike creating an impulse (a local maxima) which could then be tagged with a time stamp and compared against the previous and/or following impulse (next contact with the writing surface between strokes). This comparison will be used to create an average estimation of the user's air-time. Said algorithm is running in the background constantly and comparing its currently generated value to historical data that are read from the storage medium 30.

In view of the data generated by the two algorithms i.e., tip pressure algorithm 42 and air-time calculation algorithm 43, if the tip pressure varies significantly in the current session i.e., is reduced and the air-time has increased, the system can now generate an indication or a signal that is sent to the tactile feedback generator 18 in order to inform the user of the potential of a cognitive disorder. It should be noted that during the first sessions, the writing instrument 10 may be creating a baseline in a calibration process and it may not be capable of providing any form of feedback to the user.

A digital surface device 90 for monitoring a writing tip force of its user may also be provided to function together with a writing instrument or a pen. In this case, all or at least most of the described components are included in the digital surface device 90 while the writing instrument or pen can be a standard instrument without digital technology. Alternatively, the components shown in Fig. 1 may be distributed between the digital surface device 90 and the writing instrument.

The disclosure is based on the data that can be gathered and extrapolated from a pressure sensor that measures the forces applied by the writing instrument's tip to the writing surface. Similar benefits could be achieved by utilizing a "headless" digital surface which has no screen that can capture the pressure applied on it by a writing instrument. Said digital surface may therefore incorporate all of the other subcomponents described above and enable the same functionalities.

The digital surface device 90 may include a writing surface for writing action of the writing instrument. The writing surface may be digital or analog and includes a writing area pressure sensor 92 for monitoring or measuring the force between the writing tip of the writing instrument and a writing surface during a writing session with the writing instrument.

In embodiments, the digital surface device 90 comprises:
a writing surface configured to be written upon by a writing instrument;
one or more sensors 92 configured to monitor the force between the writing tip of the writing instrument 10 and the writing surface during a writing session with the writing instrument 10; a communication system configured to provide an indication in case that data of at least two writing sessions of the user deviates from an average of historical data from the user; and computer system configured to execute the computer-implemented method for monitoring a writing tip force of a digital writing instrument as for example depicted in Fig. 6.

The user's fingertip grip 44 depicts the physical force applied by the user due to the contact of the user's finger tips with a main body or a grip section of the writing instrument 10.

The device may also incorporate the required electronics and software drivers for connecting wirelessly (i.e., Bluetooth, Wi-Fi, etc.) to an optional supplementary device.

In order to improve the user experience and provide data to the user regarding the potential development of a cognitive disorder, the system described above can be adapted to accept input from an external mobile device, that would enable the introduction of data regarding the user's state. For example, the user can enter via a smart phone app their age, weight, gender and if they have been diagnosed with any form of Cognitive Disorder in the past.

That data can then be fed into the device's software and improve the accuracy of the system or reduce the time and/or sessions required for the device to calibrate itself and gather enough historical data.

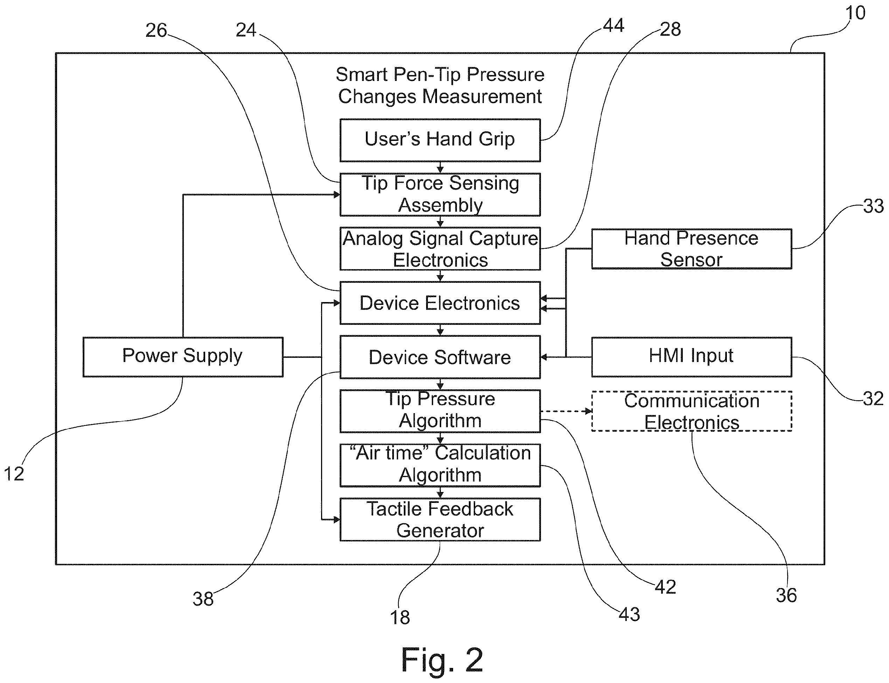

**Fig. 2** illustrates a process flow diagram of a writing instrument 10 according to the present disclosure.

The power supply 12 of the writing instrument 10 supplies power to the tip force sensing assembly 24, the device electronics 26, and the tactile feedback generator 18. The HMI input 32 supplies the device electronics 26 and the device software 38 with user input. Once a user's handgrip 44 is detected at the writing instrument 10 by a hand presence sensor 33 the tip force sensing assembly 24 detects the force and/or the pressure of the user's handgrip.

Values or parameters generated by the tip force sensing assembly 24 are received and processed by the analog signal capture electronics 28. In case of digital signals from the tip force sensing assembly 24 the analog signal capturing can be skipped. The digital signals are fed into the device electronics 26 and passed to the device software 38. Then, the digital values are input to the tip pressure algorithm 42.

The tip pressure algorithm 42 compares the measured pressure data to historical pressure data from the same user or group of users and decides whether the measured data deviates significantly from the historical data.

The air-time calculation algorithm 43 is responsible for determining the amount of time the user is spending without contacting the writing surface between two consecutive writing strokes. Said algorithm is making use of the data supplied by the tip force sensing assembly 24. Hence, the data of the tip force sensing assembly 24 in addition to the data from the hand presence sensor 33 is utilized in order to ensure that the user is actually engaging with the writing instrument 10 and it is not left on a surface for example.

If the measured and calculated data deviates significantly from the historical data by e.g., 20% the tactile feedback generator 18 is started to provide an indication to the user. Alternatively, or additionally, the outcome of the comparison is supplied to the communication electronics 36 which in turn provide the outcome of the comparison to digital device of the user.

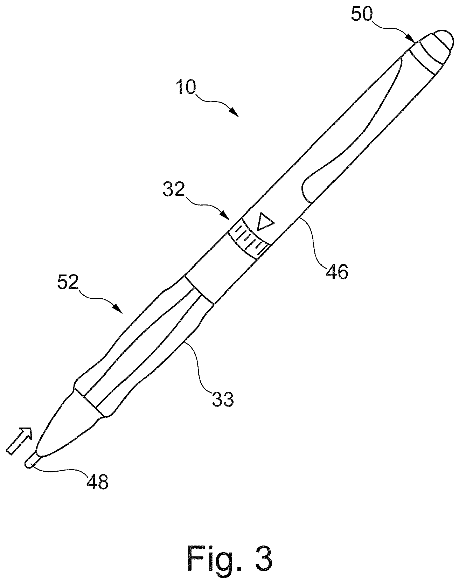

**Fig. 3** illustrates a perspective view of a writing instrument 10 according to the present disclosure. The writing instrument 10 can correspond to the writing instrument 10 as depicted in **Fig. 1** or **Fig. 2****.**

The writing instrument 10 includes a barrel shaped body 46 with a writing tip 48. The writing tip 48 could be an ink writing tip or a digital writing tip. The HMI input 32 includes in this example as selection wheel as well as the marker e.g., in form of a pointer orientated towards the selection wheel. The writing instrument 10 further includes an HMI display or HMI output 50 which is capable of indicating information like e.g., status information of the writing instrument 10 to the user. In this example, the HMI output 50 is arranged at an end opposite to the writing tip 48 and has the form of an LED strip.

The writing instrument 10 further includes a grip section 52 which is located close to the writing tip 48. At the grip section 52, the hand presence sensor 33 is located on the pen barrel close to the area that the user is making direct contact with their fingertips. Said information can be used to enhance the accuracy of the algorithm that calculates the user's air-time and/or if the device should enter a "sleep" mode.

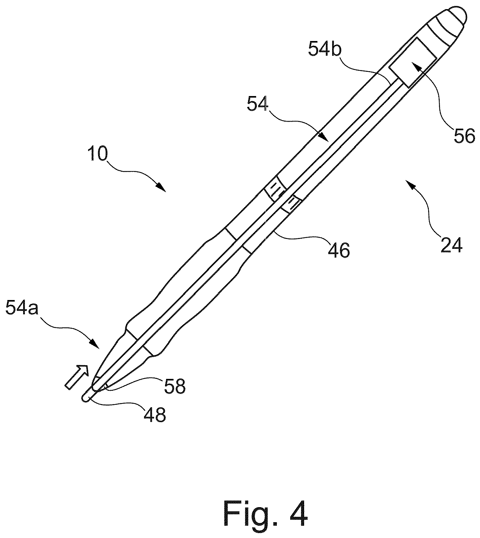

**Fig. 4** illustrates a perspective view of a writing instrument 10 according to the present disclosure. The writing instrument 10 can correspond to the writing instrument 10 as depicted in **Fig. 1** to **Fig. 3****.**

The writing instrument 10 includes an ink cartridge 54 that extends longitudinally inside the barrel shaped body 46. The ink cartridge 54 may be a hollow metal or plastic tub that store the ink. The tip force sensing assembly 24 encompasses a force sensor 56 or 58 for converting mechanical pressure into an electrical signal.

The ink cartridge 54 comprises a tip end 54a and an opposed end 54b or rear end, wherein the force sensor 56 or 58 is mechanically connected to the ink cartridge 54.

In embodiments, the ink cartridge 54 could be connected to the main body of the writing instrument 10 i.e., the barrel shaped body 46 by being press fit into the opening of the body 46. Then, the area that houses the pen tip 48 is conical and is pressed into the corresponding conical opening of the body 46. Then, the pen tip pressure is transferred via the conical attachment to the body 46.

The force sensor 58 comprises a ring of a material that can convert mechanical pressure to an electrical signal and the ring is arranged between the ink cartridge 54 and the housing or body 46. The force sensor 58 may include or consist of the conical attachment.

In embodiments, the ink cartridge 54 could be connected to the main body of the writing instrument 10 i.e., the barrel shaped body 46 by being mechanically secured between the rear end of the body 46 and a separate component that is secured press fit or via threads on the front of the pen barrel. Then, the force sensor 56 is located between the ink cartridge 54 and the rear end of the body 46.

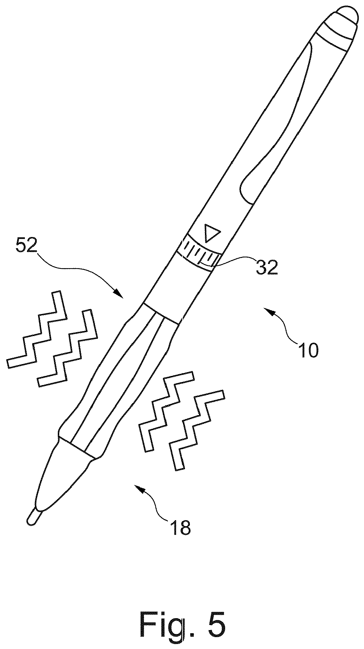

**Fig. 5** illustrates a perspective view of a writing instrument 10 according to the present disclosure. **Fig. 5** could depict different aspects of the writing instrument 10 shown in **Figs. 3** and **4**.

The writing instrument 10 may include a tactile feedback generator 18 which is arranged in the grip section 52. The tactile feedback generator 18 may be part of a communication system or is a communication system to provide an indication in case that data of at least two writing sessions of the user deviates from an average of historical data from the user. Thus, the tactile feedback generator 18 can alert the user in case of holding pressure reduction.

The user can optionally adjust the amplitude of the vibrations of the tactile feedback generator 18 via the HMI 32.

**Fig. 6** illustrates a method flow chart for monitoring a fingertip strength of a writing instrument's user according to the present disclosure.

In an exemplary step 100 of the method, the user activates their personal writing instrument 10. Such activation may be achieved by a user input e.g., via the HMI input 32. It is also possible that the writing instrument 10 automatically detects activation by the tip force sensing assembly 24 and/or the hand presence sensor 33.

In an exemplary step 110, the user initiates their note taking process or tasks as usual.

In examples and during a step 120, during writing operation, the writing instrument 10 is constantly monitoring the amount of pressure that their pen's tip is applying on the writing surface.

To that end, force sensors are capturing accurate data from the interaction between the user's hand, the pen's barrel, the ink cartridge (pen's nib) and the writing surface. In the background, the writing instrument 10 is comparing the newly captured and extrapolated data to historical ones from the same user.

In other words, the step 120 includes generating data from the monitored force, storing generated data as historical data, and comparing the generated data to historical data from the user.

In an exemplary step 130, the captured data is extrapolated to evaluate the time spent between two consecutive writing strokes.

In other words, the step 130 includes to extrapolate a time span of the writing instrument not in contact with the writing surface between two consecutive writing strokes from the monitored force; and to generate data from the monitored force and the extrapolated time span.

In an exemplary step 140, in the event that multiple writing sessions from the user have indicated that pressure applied on the writing surface has varied significantly and the air-time has increased, the pen will alert them. Reduction in pen tip pressure while writing can be associated with increased risk of dementia or cognitive disorders. The increase of air-time can indicate the potential of a cognitive disorder.

In other words, the step 140 includes providing an indication in case that data of at least two writing sessions of the user deviates from an average of historical data from the user.

An indication may be provided in case the force is below an average force of historical data and/or a time span of the writing instrument 10 not in contact with the writing surface between two consecutive writing strokes is above an average time span of historical data.

In an exemplary sixth step 150, a tactile feedback generator communicates to the user the alert or the indication and may inform the user to seek medical attention. In examples, the user may adjust the amplitude of the vibrations of the feedback generator via the HMI input 32.

At least one step of this method is executed by the writing instrument 10. Additionally, or alternatively, at least one step of this method is executed by the digital surface device 90 comprising the writing surface.

The present disclosure also relates to the computer-implemented method for monitoring a fingertip strength of a writing instrument's user and the writing instrument for monitoring a fingertip strength of its user of the following aspects:
1. A computer-implemented method for monitoring a writing tip force of a writing instrument, comprising:
   - monitoring the force between the writing tip of the writing instrument and a writing surface during a writing session with the writing instrument;
   - generating data from the monitored force;
   - storing generated data as historical data
   - comparing the generated data to historical data from the user; and
   - providing an indication in case that data of at least two writing sessions of the user deviates from an average of historical data from the user.
2. The method of aspect 1, comprising:
   - extrapolate a time span of the writing instrument not in contact with the writing surface between two consecutive writing strokes from the monitored force; and
   - generating data from the monitored force and the extrapolated time span.
3. The method of one of the preceding aspects, wherein at least one step of the method is executed by the writing instrument.
4. The method of one of the preceding aspects, wherein at least one step of the method is executed by a digital surface device comprising the writing surface.
5. The method of one of the preceding aspects, wherein an indication is provided in case the force is below an average force of historical data and/or a time span of the writing instrument not in contact with the writing surface between two consecutive writing strokes is above an average time span of historical data.
6. The method of one of the preceding aspects, comprising:
   - providing personal user data for calibration and/or comparing the generated data to historical data from the user.
7. The method of one of the preceding aspects, comprising:
   - alerting the user in case of an indication by activating a tactile feedback generator of the writing instrument.
8. The method of one of the preceding aspects, comprising:
   - providing haptic feedback to the user by activating a tactile feedback generator of the writing instrument.
9. The method of one of the preceding aspects, wherein the indication is provided via a user interface of the writing instrument or wherein the indication is provided via a wireless communication system to an external device having an interface.
10. A writing instrument for monitoring a writing tip force of its user, comprising:
   - one or more sensors configured to monitor the force between the writing tip of the writing instrument and a writing surface during a writing session with the writing instrument;
   - a communication system configured to provide an indication in case that data of at least two writing sessions of the user deviates from an average of historical data from the user;
   - a storage system configured to store generated data as historical data; and
   - a computer system configured to execute the computer-implemented method for monitoring a writing tip force of a digital writing instrument according to one of the preceding aspects.
11. The writing instrument of aspect 10, comprising:
   - a hand presence sensor configured to obtain information regarding a usage state of the writing instrument.
12. The writing instrument of aspect 10 or 11, comprising:
   - a tactile feedback generator configured to alert the user in case that data of at least two writing sessions of the user deviates from an average of historical data from the user.
13. The writing instrument of one of aspects 10 to 12, comprising:
   - a tactile feedback generator configured to provide haptic feedback to the user.
14. The writing instrument of aspect 12 or 13, wherein the tactile feedback generator is configured to generate vibrations in the range of 0 to 500Hz with a controllable amplitude.
15. The writing instrument of one of aspects 10 to 14, comprising:
   - an ink cartridge comprising a tip end and an opposed end, wherein the one or more sensors are mechanically connected to the ink cartridge.
16. The writing instrument of aspects 15, comprising:
   - a barrel-shaped housing, wherein the one or more sensors are arranged between the opposed end of the ink cartridge and the housing.
17. The writing instrument of aspect 15 or 16, comprising:
   - a barrel-shaped housing, wherein the one or more sensors comprise a ring of a material that can convert mechanical pressure to an electrical signal and wherein the ring is arranged between the ink cartridge and the housing.
18. The writing instrument of one of aspects 10 to 17, comprising:
   - a user interface configured to receive input from a user and/or to provide information to the user such as the indication.
19. The writing instrument of one of aspects 10 to 18, comprising:
   - a wireless communication system configured to provide the indication via an external device having an interface.
20. A digital surface device for monitoring a writing tip force of its user, comprising:
   - a writing surface configured to be written upon by a writing instrument;
   - one or more sensors configured to monitor the force between the writing tip of the writing instrument and the writing surface during a writing session with the writing instrument;
   - a communication system configured to provide an indication in case that data of at least two writing sessions of the user deviates from an average of historical data from the user; and
   - a computer system configured to execute the computer-implemented method for monitoring a writing tip force of a digital writing instrument according to one of the aspects 1 to 9.
21. A writing instrument for monitoring a writing tip force of its user, comprising:
   - a barrel shaped body and a two or more of magnets that are housed inside the barrel shaped body wherein a first magnet is fixed to an end of the writing tip of the writing instrument and a second magnet is permanently fixed to a wall inside the barrel shaped body,
   - a communication system configured to provide an indication in case that data of at least two writing sessions of the user deviates from an average of historical data from the user;
   - a storage system configured to store generated data as historical data; and
   - a computer system configured to execute the computer-implemented method for monitoring a writing tip force of a digital writing instrument according to aspects 1-9.
22. A digital surface device for monitoring a writing tip force of its user, comprising:
   - a writing surface configured to be written upon by a writing instrument (10) according to aspect 21;
   - a plurality of magnetometers that are mechanically linked to one another,
   - a communication system configured to provide an indication in case that data of at least two writing sessions of the user deviates from an average of historical data from the user; and
   - a computer system configured to execute the computer-implemented method for monitoring a writing tip force of a digital writing instrument (10) according to aspects 1-9.

## Claims

1. A computer-implemented method for monitoring a writing tip force of a writing instrument (10), comprising:
- monitoring the force between the writing tip (48) of the writing instrument (10) and a writing surface during a writing session with the writing instrument (10);
- generating data from the monitored force;
- storing generated data as historical data
- comparing the generated data to historical data from the user; and
- providing an indication in case that data of at least two writing sessions of the user deviates from an average of historical data from the user.

2. The method of claim 1, comprising:
- extrapolate a time span of the writing instrument (10) not in contact with the writing surface between two consecutive writing strokes from the monitored force; and
- generating data from the monitored force and the extrapolated time span.

3. The method of one of the preceding claims, wherein at least one step of the method is executed by the writing instrument (10).

4. The method of one of the preceding claims, wherein at least one step of the method is executed by a digital surface device (90) comprising the writing surface.

5. The method of one of the preceding claims, wherein an indication is provided in case the force is below an average force of historical data and/or a time span of the writing instrument (10) not in contact with the writing surface between two consecutive writing strokes is above an average time span of historical data.

6. The method of one of the preceding claims, comprising:
- providing personal user data for calibration and/or comparing the generated data to historical data from the user.

7. The method of one of the preceding claims, comprising:
- alerting the user in case of an indication by activating a tactile feedback generator (18) of the writing instrument (10).

8. The method of one of the preceding claims, wherein the indication is provided via a user interface of the writing instrument (10) or wherein the indication is provided via a wireless communication system (36) to an external device (90) having an interface.

9. A writing instrument (10) for monitoring a writing tip force of its user, comprising:
- one or more sensors (56, 58) configured to monitor the force between the writing tip (48) of the writing instrument (10) and a writing surface during a writing session with the writing instrument (10);
- a communication system (36) configured to provide an indication in case that data of at least two writing sessions of the user deviates from an average of historical data from the user;
- a storage system (30) configured to store generated data as historical data; and
- a computer system (34) configured to execute the computer-implemented method for monitoring a writing tip force of a digital writing instrument (10) according to one of the preceding claims.

10. The writing instrument (10) of claim 9, comprising:
- a hand presence sensor (33) configured to obtain information regarding a usage state of the writing instrument (10).

11. The writing instrument (10) of claim 9 or 10, comprising:
- a tactile feedback generator (18) configured to alert the user in case that data of at least two writing sessions of the user deviates from an average of historical data from the user.

12. The writing instrument (10) of one of claims 9 to 11, comprising:
- an ink cartridge (54) comprising a tip end (54a) and an opposed end (54b), wherein the one or more sensors (56, 58) are mechanically connected to the ink cartridge (54).

13. The writing instrument (10) of claims 12, comprising:
- a barrel-shaped housing (46), wherein the one or more sensors (56) are arranged between the opposed end (54b) of the ink cartridge (54) and the housing (46).

14. The writing instrument (10) of claim 12 or 13, comprising:
- a barrel-shaped housing (46), wherein the one or more sensors (58) comprise a ring of a material that can convert mechanical pressure to an electrical signal and wherein the ring is arranged between the ink cartridge (54) and the housing (46).

15. A digital surface device (90) for monitoring a writing tip force of its user, comprising:
- a writing surface configured to be written upon by a writing instrument (10) according to claims 9-14;
- one or more sensors (92) configured to monitor the force between the writing tip (48) of the writing instrument (10) and the writing surface during a writing session with the writing instrument (10);
- a communication system configured to provide an indication in case that data of at least two writing sessions of the user deviates from an average of historical data from the user; and
- a computer system configured to execute the computer-implemented method for monitoring a writing tip force of a digital writing instrument (10) according to one of the claims 1 to 8.
